# EUROPEAN PATENT APPLICATION

(11) **EP 0 747 077 A2**
(43) Date of publication of application: **11.12.1996**
(21) Application number: 96304249.4
(22) Date of filing: 07.06.1996
(51) Int. Cl.: A61M 16/04

(54) **Endotracheal tube**

(30) Priority: 07.06.1995 ZA 9504682
(71) Applicant: Raw, Robert Maurice, Johannesburg, Transvaal Province (ZA)
(72) Inventor: Raw, Robert Maurice, Johannesburg, Transvaal Province (ZA)
(74) Representative: Howick, Nicholas Keith

(57) **Abstract**

This invention relates to an tracheostomy tube which reduces considerably apparatus dead-space. The tube (1) has an inspiratory gas passageway (2) which is connectable to anaesthesia and/or ventilatory machinery, a separate expiratory gas passageway (3) and an inflatable cuff (6). It is envisaged that in separating the inspiratory and expiratory passageways apparatus dead space is reduced or eliminated.

## Description

### INTRODUCTION

THIS INVENTION relates to an endotracheal tube and more particularly to an endotracheal tube which eliminates or at least reduces considerably dead space within the tube.

### BACKGROUND TO THE INVENTION

Endotracheal tubes are used in certain medical procedures to ventilate patients. They are usually used during surgical procedures, post-surgical recuperation and in intensive or high care wards.

The tubes are inserted through the oral or nasal passageways or through a surgically created canal into the trachea and into the trachea of an individual to stop short a short distance from the division of the trachea into bronchi. Once correctly positioned a cuff located towards the operatively lower end of the tube is inflated to seal the gap between the trachea and tube walls and also to at least partly retain the tube within the trachea.

The operatively outer end of the tube projects from the patient's mouth or nares or from the surgically created canal and at some point in the system, is branched forming an exhaust passageway for expired air, and an inlet passageway for inspired air. The inlet passageway is connected to a gas regulator which receives and mixes various anaesthetic and respiratory gasses. These gasses are inspired by the patient via the lumen of the tube. A valve means prevents air being drawn into the tube through the exhaust passageway.

On expiration expired air passes up the lumen of the tube and exhausts passed the valve means to atmosphere.

Another type of tube known to the applicant is an endobronchial tube. This type of tube has two lumens which are fused for the greater part of their length. One lumen, the bronchial lumen projects a short distance beyond the end of the other lumen, the tracheal lumen. This tube is positioned such that the bronchial lumen or tube is located within the bronchus of one lung while the tracheal lumen or tube is located within the trachea. Inflatable cuffs seal the gaps between the tubes and trachea and bronchus. By stopping the flow to and from one of the tubes, either lung of a patient can be ventilated enabling surgical procedures to be conducted on the other lung. As with the single lumen endobronchial tube, each tube carries inspired and expired air.

Still another type of endotracheal tube is also termed a tracheotomy tube and is inserted through a surgically created canal in a patents trachea to facilitate breathing where the normal passages are occluded. As used in this specification, the term endotracheal tube is intended to include this so-called tracheotomy tube.

The above described endotracheal and endobronchial tubes have a disadvantage in that they use a single tube to convey both inspired and expired air. This is a disadvantage because the length of the tube or tubes creates a dead space. Thus expired air in this dead space is reintroduced into the lungs of a patient before inspired air. The disadvantages of this are clear to persons skilled in the art namely specialists in the field of anaesthesia and include a build up of carbon dioxide and increased usage of anaesthetic gasses. The former is usually countered by increasing the volume of inspiratory gasses pumped into a patient's lungs. This in turn also results in increased usage of anaesthetic gasses and has several other disadvantages including:
1. increased diaphragmatic movement;
2. increased peak inspiratory pressure;
3. increased mean airway pressure; and
4. increased work in breathing which is important when a patient is to be weaned from a ventilator.

It must be understood that the term "air" when used in this specification is intended to include air and mixtures of gasses usually used for medical purposes.

### OBJECT OF THE INVENTION

It is an object of this invention to provide an endotracheal tube which at least partly alleviates the above mentioned disadvantages experienced with conventional endotracheal tubes.

### SUMMARY OF THE INVENTION

In accordance with this invention there is provided an endotracheal tube comprising at least one discrete inspiratory passageway and at least one discrete expiratory passageway, the tube having an operatively inner end which is locatable within an individual's trachea and at least one inflatable cuff located towards the operatively inner end of the tube.

There is also provided for the endotracheal tube to have an operatively outer end which projects, in use, from an individual's oral or nasal passages. Alternatively there is provided for the endotracheal tube to have an operatively outer end which projects, in use, from a surgically created canal in an individual's throat.

There is also provided for the inspiratory and expiratory passageways to be formed by separate subsidiary tubes, and for the subsidiary tubes to be joined together at at least their operatively inner ends. Alternatively there is provided for the endotracheal tube to be a single tube having a longitudinal divider forming at least one pair of lumens which in turn form the inspiratory passageway and the expiratory passageway.

There is further provided for the operatively outer end of the endotracheal tube to be branched to form an inspiratory branch which is connected to the inspiratory passageway and an expiratory branch which is connected to the expiratory passageway, and for the branches to be connectable to conventional ventilation and/or anaesthesia apparatuses.

There is further provided for the inflatable cuff to be connected to a pneumatic tube which is integral with and which extends along the length of the endotracheal tube.

Further features of the invention provide for the endotracheal tube to be manufactured from a synthetic plastics material, preferably polyvinyl chloride; for the tube to be oval in cross section having a major external dimension of approximately 16,5mm and a minor external dimension of approximately 16,0mm; and for the endotracheal tube to be approximately 280mm long.

### BRIEF DESCRIPTION OF THE DRAWINGS

Preferred embodiments of the invention will be described below by way of example only and with reference to the accompanying drawings in which:
- Figure 1: is a sectional side view of a first embodiment of an endotracheal tube according to the invention;
- Figure 2: is a cross sectional plan view of the endotracheal tube of Figure 1 taken along section A-A;
- Figure 3: is a side elevational view of a second embodiment of an endotracheal tube according to the invention;
- Figure 4: is a side elevational view of a third embodiment of an endotracheal tube according to the invention;
- Figure 5: is a side elevational view of a fourth embodiment of an endotracheal tube according to the invention; and
- Figure 6: is a cross sectional plan view corresponding to the view in Figure 2 of a fifth embodiment of an endotracheal tube according to the invention.

### DETAILED DESCRIPTION OF THE DRAWINGS

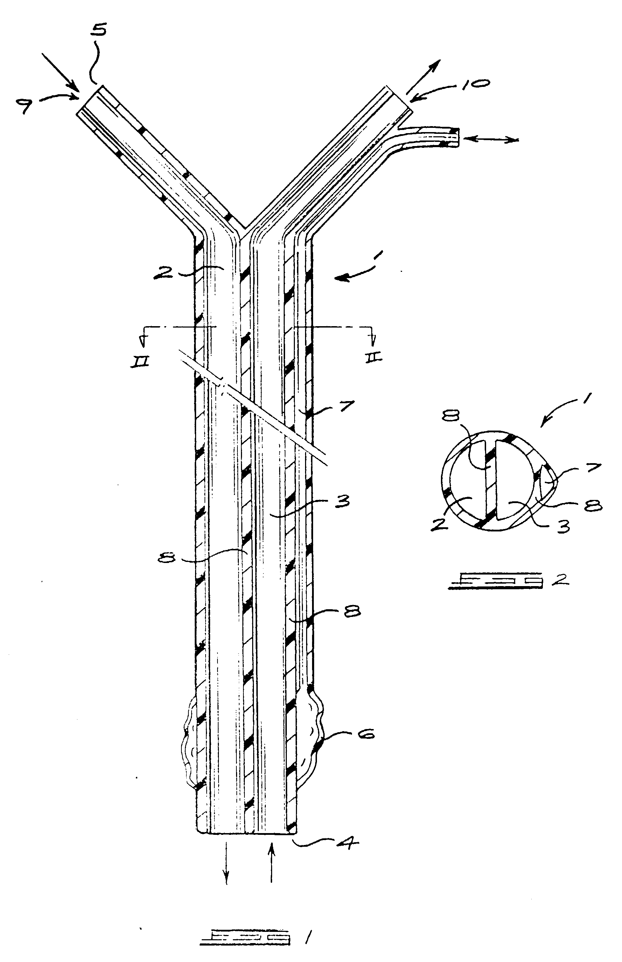

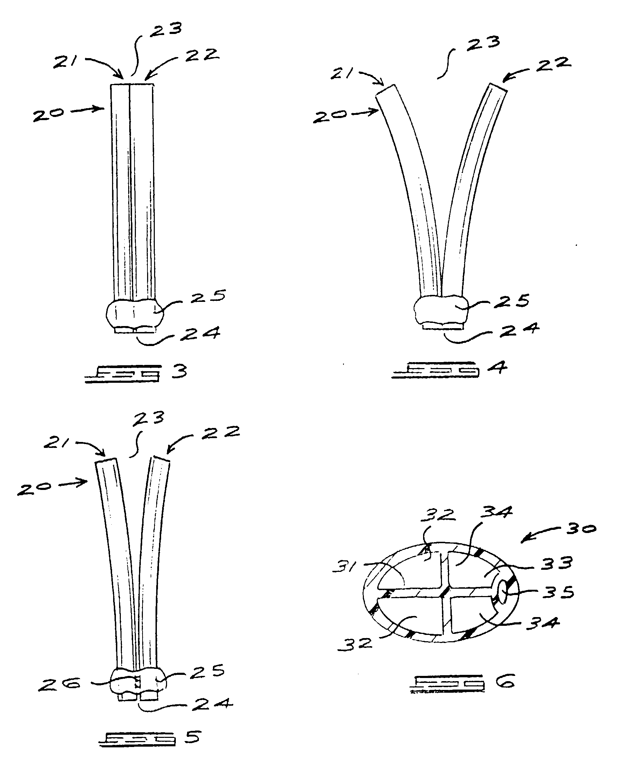

Referring to Figures 1 and 2, an endotracheal tube (1) comprises a single discrete inspiratory passageway (2) and a single discrete expiratory passageway (3). The tube (1) has an operatively inner end (4) which is locatable within an individual's trachea (not shown) and an operatively outer end (5) which projects, in use, from said individual's oral or nasal passages (not shown). An inflatable cuff (6) is located towards the operatively inner end (4) of the tube (1). The cuff (6) is inflated, in use, after the tube (1) is positioned in an individual's trachea by pumping air down a pneumatic tube (7).

The endotracheal tube (1) is manufactured from polyvinyl chloride and is oval in cross section. Longitudinal dividers (8) run the length of the tube (1) to separate the inspiratory passageway (2), expiratory passageway (3) and pneumatic tube (7).

The operatively outer end (5) of the endotracheal tube (1) is branched to form an inspiratory branch (9) and an expiratory branch (10). These branches (9 & 10) are connectable, in use, to conventional anaesthesia or ventilation machines.

In the embodiment illustrated, the tube (1) is approximately 280mm in length. Its major diameter is approximately 16,5mm and its minor diameter is approximately 16,0mm. The tube has approximately 1,5mm thick walls and dividers and thus the dimensions of the inspiratory and expiratory passageways are approximately 6,0mm by 13,0mm. From the above dimensions it will be appreciated that the above described endotracheal tube is inserted via the oral passageway of an individual. Should these dimensions be scaled down however the tube can be inserted via the nasal passageways.

Referring to Figures 3, 4 and 5, alternate embodiments of endotracheal tubes (20) are illustrated. Each of these tubes has a discrete inspiratory passageway (21) and a discrete expiratory passageway (22). Each passageway has an operatively upper end (23) and an operatively lower end (24). An inflatable cuff (25) is located around the tubes forming the passageways towards the operatively lower end (24).

Referring specifically to Figure 3, the inspiratory tube (21) and expiratory tube (22) are connected to each other along the whole of their length. Referring specifically to Figures 4 and 5, the inspiratory tube (21) and expiratory tube (22) are connected to each other only towards their operatively inner ends (24). The embodiment illustrated in Figure 4 has the tubes welded to each other while the tubes of the embodiment illustrated in Figure 5 has the tubes connected by a connector element (26).

Referring to Figure 6, a further embodiment of an endotracheal tube (30) is illustrated. In this embodiment the inspiratory passageway (31) is formed by a pair of lumens (32). The expiratory passageway (33) is also formed by a pair of lumens (34). The tube (30) is a single polyvinyl chloride moulding and includes a pneumatic tube (35) for inflating an inflatable cuff (not shown).

Referring now to Figure 1, in use, an endotracheal tube (1) is inserted into the trachea of an individual (not shown) via the oral passageway. Once correctly positioned with its operatively inner end (4) as few centimetres above the bronchial branch, the inflatable cuff (6) is inflated with a small pump which could be a syringe. A non-return valve (not shown) is fitted to the open end of the pneumatic tube (7) to prevent accidental deflation of the cuff (6). When inflated, the cuff effectively seals any openings between the tube (1) and the walls of the trachea. It also centres the tube (1) in the trachea and, to an extent, prevents its accidental withdrawal.

After positioning, a ventilator and anaesthetic gas regulator (not shown) are connected to the inspiratory branch (9) of the tube (1). The expiratory branch (10) can be connected to an expired gas scrubber or fitted with a non-return valve which allows gas to egress the branch (10) only.

Anaesthetic gas, typically a mixture of gasses, can now be pumped into the lungs of an individual or drawn into the lungs by normal respiration where the individual is capable of normal respiration. Expired gas is drawn from the lungs or is breathed out and exits the lungs via the expiratory branch (3). Thus there is no mixing of inspired gas and expired gas within the endotracheal tube (1) and the only dead space is in the trachea, bronchi and lungs themselves.

It is envisaged that this reduction or elimination of apparatus dead space has numerous advantages over conventional endotracheal tubes. There is a considerably reduction in rebreathed carbon dioxide which, of necessity, is reinhaled. This in turn reduces the overall carbon dioxide concentration in the lungs of an individual which enhances diffusion of carbon dioxide from the blood into the lungs from where it is exhaled. Because of this the volume of inhaled gas can be reduced considerably while maintaining carbon dioxide elimination.

It is also envisaged that reducing the volume of inhaled gas will reduce the costs of anaesthesia which may be of considerable importance in underdeveloped countries where financial resources and medical supplies are limited.

A further possible advantage of endotracheal tubes according to the invention is in a new approach to pulmonary afflictions. In one such affliction, adult respiratory distress syndrome or ARDS, patients are ventilated and ventilatory pressures can cause trauma to an already traumatised lung. The reduction in inspired gas volume reduces ventilatory pressures which reduces secondary trauma. The same holds true for persons suffering from acute asthma, emphysema and bulbous lung disease.

It will be appreciated that many variations can be made to the above described embodiments of the invention without departing from the scope thereof. In particular inspired and/or expired gas sampling tubes may be incorporated into the endotracheal tube. The tube itself may be a single use disposable item or may be sterilisable for multi use applications. The tube may also be generally straight or curved and it may be rigid or flexible.

## Claims

1. An endotracheal tube having an operatively inner end which is locatable within an individual's trachea and at least one inflatable cuff located towards the operatively inner end of the tube characterised in that said tube has at least one discrete inspiratory passageway and at least one discrete expiratory passageway.

2. An endotracheal tube as claimed in claim 1 characterised in that the tube has a second end which projects, in use, from an individual's oral or nasal passages.

3. An endotracheal tube as claimed in claim 1 characterised in that the tube has a second end which projects, in use, from a surgically created canal in an individual's throat.

4. An endotracheal tube as claimed in claim 2 or in claim 3 characterised in that the inspiratory and expiratory passageways are formed by separate subsidiary tubes.

5. An endotracheal tube as claimed in claim 4 characterised in that the subsidiary tubes are joined together at at least their operatively inner ends.

6. An endotracheal tube as claimed in claim 2 or in claim 3 characterised in that the endotracheal tube is a single tube having a longitudinal divider forming at least one pair of lumens which in turn form the inspiratory passageway and the expiratory passageway.

7. An endotracheal tube as claimed in claim 6 characterised in that the operatively outer end of the endotracheal tube is branched to form an inspiratory branch which is connected to the inspiratory passageway and an expiratory branch which is connected to the expiratory passageway.

8. An endotracheal tube as claimed claim 6 or in claim 7 characterised in that the inflatable cuff is connected to a pneumatic tube which is integral and runs along the length of the endotracheal tube.

9. An endotracheal tube as claimed in any one of claims 1 to 8 characterised in that the endotracheal tube is manufactured from a synthetic plastics material.

10. An endotracheal tube as claimed in claim 9 characterised in that the synthetic plastics material is polyvinyl chloride.

11. An endotracheal tube as claimed in claim 6 characterised in that the tube is oval in cross section and has a major external dimension of approximately 16,5mm and a minor external dimension of approximately 16,0mm.

12. An endotracheal tube as claimed in claim 11 characterised in that the endotracheal tube is approximately 280mm long.
